(19)

Europäisches
Patentamt
European
Patent Office
Office européen
des brevets

(11)  **EP 1 783 208 A1**

(12)  **EUROPEAN PATENT APPLICATION**
published in accordance with Art. 158(3) EPC

(43) Date of publication:
**09.05.2007  Bulletin 2007/19**

(21) Application number: **05757788.4**

(22) Date of filing: **06.07.2005**

(51) Int Cl.:
***C12N 5/06*** (2006.01)

(86) International application number:
**PCT/JP2005/012472**

(87) International publication number:
**WO 2006/004149 (12.01.2006 Gazette 2006/02)**

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **06.07.2004  JP  2004199609**

(71) Applicants:
  • **KYOWA HAKKO KOGYO CO., LTD.
    Chiyoda-ku,
    Tokyo 100-8185 (JP)**
  • **Iwata, Hiroo
    Kyoto-shi, Kyoto 606-8507 (JP)**
  • **Sasai, Yoshiki
    Hyogo 650-0047 (JP)**

(72) Inventors:
  • **IWATA, Hiroo
    c/o Institute for Frontier Med. Sc.
    Kyoto-shi, Kyoto 606 8507 (US)**

  • **SASAI, Yoshiki
    c/o Center for Development Biology
    Hyogo 650-0047 (JP)**
  • **YAMAZOE, Hironori
    c/o Nanotechnology Research Inst
    Ibaraki 305-8566 (JP)**
  • **KOBORI, Masato
    c/o BioFrontier Laboratories
    Tokyo 194-8533 (JP)**
  • **SATOH, Mitsuo
    c/o BioFrontier Laboratories
    Tokyo 194-8533 (JP)**
  • **YANO, Keiichi
    c/o BioFrontier Laboratories
    Tokyo 194-8533 (JP)**

(74) Representative: **Vossius & Partner
    Siebertstrasse 4
    81675 München (DE)**

(54)  **METHOD OF PRODUCING NERVE CELL**

(57)    An Object of the present invention is to provide a process for producing a nerve cell by inducing differentiation of an embryonic stem cell, a method for inducing differentiation of the embryonic stem cell into a nerve cell, a medium to be used in the production process or differentiation induction method, or a method for improving purity of the nerve cell obtained by inducing differentiation of the embryonic stem cell. The present invention provides a process for producing a nerve cell which is applicable to treatment of neurodegenerative disease or the like easily, selectively or inexpensively by inducing differentiation induction of an embryonic stem cell using vitamin $B_{12}$ or a salt thereof and heparin, a substance having heparin-like activity or a salt.

EP 1 783 208 A1

**Description**

TECHNICAL FIELD

The present invention relates to a process for producing a nerve cell by inducing differentiation of an embryonic stem cell, a method for inducing differentiation of the embryonic stem cell into a nerve cell, a medium used in the production process and the differentiation induction method, and a method for improving purity of the nerve cell obtained by inducing differentiation of the embryonic stem cell.

BACKGROUND ART

Parkinson's disease is a neurodegenerative disease which is caused by the degeneration and loss of dopaminergic neurons existingin the midbrain substantia nigra and which causes dyskinesia, such as tremor, rigidity and bradykinesia, due to reduction of dopamine in the corpus striatum. As the therapeutic method, administration of a dopamine precursor L-DOPA (L-dihydroxyphenylalanine) is often used for the purpose of supplementing the dopamine in the corpus striatum, but attenuation of its effect is observed resulting from the long-term administration or advance of symptoms. A cell transplantation treatment has been attempted for such patients of severe Parkinson's disease (cf., Non-patent literature 1), but it is difficult in reality to keep a large amount of cells for use in the transplantation.

The embryonic stem cell, also called ES cell, is a cell which can be cultured *in vitro* and can be differentiated into all cells including reproductive cells when injected into the vacuole of an embryo of other individual before implantation, such as a blastocyst. In order to obtain the cells to be transplanted into patients of Parkinson's disease, various attempts have been made on the differentiation induction of the ES cell into dopaminergic neuron.

Lee *et al.* reported that a dopaminergic neuron can be obtained by selecting and proliferating nestin-positive cells after forming a cell mass (embryoid bodies) from ES cells (cf., Non-patent literature 2). Also, Kawasaki *et al.* reported that a dopaminergic neuron can be obtained within a short period of time when ES cells are cultured using mouse-derived PA6 cells as feeder cells (cf., Non-patent literature 3). It is shown that a proteinous factor having activity of inducing differentiation of embryonic stem cells into ectodermal cells or ectoderm-derived cells (stromal cell-derived inducing activity) has been identified from the feeder cells, and that a dopaminergic neuron is induced from the ES cells when the proteinous factor is added to a medium (cf., Patent literature 1). In addition, it was reported that dopaminergic neuron can be induced when a nuclear receptor Nurr1 (cf., Non-patent literature 4) or a Wnt antagonist SFRP-2 (cf., Non-patent literature 5) is over-expressed in ES cells.

It has been known that vitamin $B_{12}$ has pharmacological activity such as restoration of peripheral nerve tissues by accelerating metabolism of nucleic acid, protein and lipid through its transmethylation reaction. On the other hand, a group of sulfated mucopolysaccharides, so-called heparin, are used in the prevention and treatment of thromboembolism, and it has been reported recently that a low molecular heparin has activity related to the existence and growth of motor neuron (cf., Patent literature 2). It is known that polyamines such as biotin and putrescine and iron-containing compounds are components generally contained in the media for carrying out culturing of various microorganisms, plant cells, animal cells and the like, and show various activities such as metabolism accelerating activity and protein and nucleic acid synthesis accelerating activity through their role as a vitamin, coenzyme, polyamine or trace nutrient substance. Biotin (vitamin H) plays an essential role in the carboxylation reaction and decarboxylation reaction by an enzyme catalyst and is an essential factor in viable cells, because almost all animals cannot synthesize biotin by themselves and therefore have to incorporate biotin from the outside world. Regarding polyamine, its activity of accelerating elongation of axon process of rat cultured hippocampus nerve cells is known (cf., Non-patent literature 6).

Patent literature 1:       WO03/42384 pamphlet
Patent literature 2:       Japanese Published Unexamined Patent Application No. 2002-53243
Non-patent literature 1:   New England Journal of Medicine, 344, 710 (2001)
Non-patent literature 2:   Nature Biotechnology, 18, 675 (2000)
Non-patent literature 3:   Proceedings of the National Academy of Sciences of the United States of America, 99, 1580 (2002)
Non-patent literature 4:   Nature, 418, 50 (2002)
Non-patent literature 5:   Nature Biotechnology, 20, 1240 (2002)
Non-patent literature 6:   Neuroscience Research, 19, 155 (1994)

DISCLOSURE OF THE INVENTION

Problems to be solved by the invention:

Objects of the present invention are to provide a process for producing a nerve cell by inducing differentiation of an embryonic stem cell, a method for inducing differentiation of the embryonic stem cell into a nerve cell, a medium to be used in the production process or differentiation induction method, or a method for improving purity of the nerve cell obtained by inducing differentiation of the embryonic stem cell.

Means for solving the problems:

The present invention relates to the following (1) to (16).

(1) A process for producing a nerve cell, which comprises steps of culturing an embryonic stem cell under non-aggregation conditions using vitamin $B_{12}$ or a salt thereof and heparin, a substance having heparin-like activity or a salt thereof to thereby induce differentiation into a nerve cell, and isolating a nerve cell from the culture.
(2) The process according to the above-described (1), wherein the nerve cell is a catecholaminergic neuron.
(3) The process according to the above-described (2), wherein the catecholaminergic neuron is a dopaminergic neuron.
(4) The process according to any one of the above-described (1) to (3), wherein the differentiation induction is carried out in a serum-free medium.
(5) The process according to the above-described (4), wherein the differentiation is induced without utilizing such activity owned by a stroma cell that induces differentiation of an embryonic stem cell into an ectodermal cell or an ectoderm-derived cell.
(6) The process according to the above-described (4) or (5), wherein the serum-free medium is a serum-free medium which comprises one or more compounds selected from biotin or a salt thereof, a polyamine and an iron-containing compound.
(7) A medium having activity of inducing differentiation of an embryonic stem cell into a nerve cell, which is used for the process described in any one of the above-described (1) to (6).
(8) A method for inducing differentiation of an embryonic stem cell into a nerve cell, which comprises a step of culturing the embryonic stem cell under non-aggregation conditions using vitamin $B_{12}$ or a salt thereof and heparin, a substance having heparin-like activity or a salt thereof.
(9) The method according to the above-described (8), wherein the nerve cell is a catecholaminergic neuron.
(10) The method according to the above-described (9), wherein the catecholaminergic neuron is a dopaminergic neuron.
(11) The method according to any one of the above-described (8) to (10), wherein the differentiation induction is carried out in a serum-free medium.
(12) The method according to the above-described (11), wherein the differentiation is induced without utilizing such activity owned by a stroma cell that induces differentiation of an embryonic stem cell into an ectodermal cell or an ectoderm-derived cell.
(13) The method according to the above-described (11) or (12), wherein the serum-free medium is a serum-free medium which comprises one or more compounds selected from biotin or a salt thereof, a polyamine and an iron-containing compound.
(14) A medium having activity of inducing differentiation of an embryonic stem cell into a nerve cell, which is used for the method described in any one of the above-described (8) to (13).
(15) A method for improving purity of a nerve cell obtained by inducing differentiation of an embryonic stem cell, which comprises steps of culturing the embryonic stem cell under non-aggregation conditions using vitamin $B_{12}$ or a salt thereof and heparin, a substance having heparin-like activity or a salt thereof, and then culturing in a medium containing an anticancer agent.
(16) The method according to the above-described (15), wherein the anticancer agent is an anticancer agent selected from the group consisting of mitomycin C, 5-fluorouracil, adriamycin, Ara-C and methotrexate.

Effect of the invention:

In the present invention, a process for producing a nerve cell by inducing differentiation of an embryonic stem cell, a method for inducing differentiation of the embryonic stem cell into a nerve cell, a medium to be used in the production process or differentiation induction method, or a method for improving purity of the nerve cell obtained by inducing differentiation of the embryonic stem cell is provided.

In the present invention, differentiation of an embryonic stem cell into a dopaminergic neuron can be induced conveniently, selectively and inexpensively, without utilizing a specific proteinous factor, and also without carrying out complex operations such as multistage culturing, co-culturing with a heterologous cell and gene manipulation for inducing the differentiation toward a specific direction.

BRIEF DESCRIPTION OF THE DRAWINGS

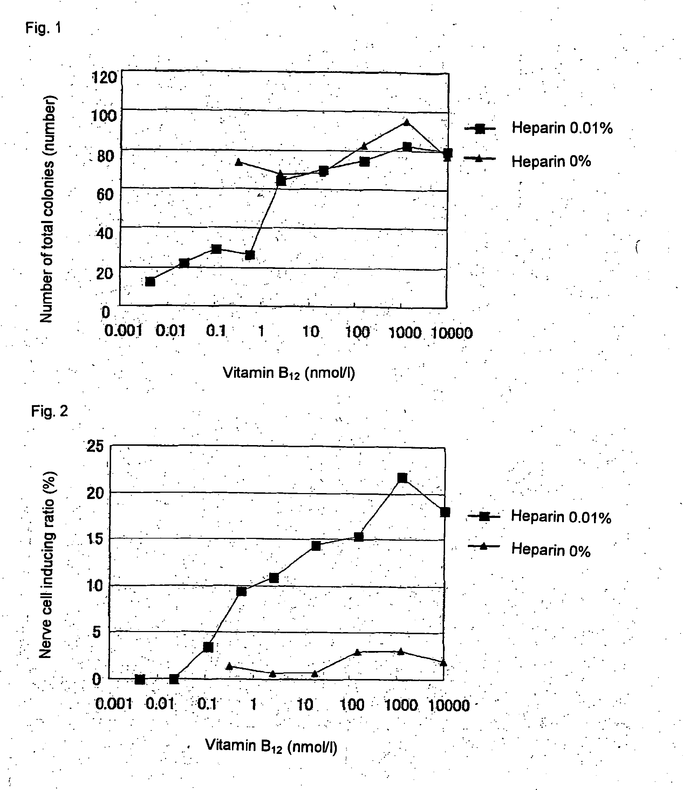

Fig. 1 is a graph showing the number of colonies formed from EB5 when concentrations of heparin and vitamin $B_{12}$ were changed. In the graph, ■ shows 0.01% heparin addition conditions, and ▲ shows 0% heparin addition conditions. Also, in the graph, the abscissa shows the concentration of vitamin $B_{12}$ (nmol/l) contained in the test solution 1, and the ordinate shows the number of total colonies observed 14 days after commencement of the culturing.

Fig. 2 is a graph showing the ratio of nerve cells induced from EB5, namely nerve cell inducing ratio, when concentrations of heparin and vitamin $B_{12}$ were changed. In the graph, ■ shows 0.01% heparin addition conditions, and ▲ shows 0% heparin addition conditions. Also, in the graph, the abscissa shows the concentration of vitamin $B_{12}$ (nmol/l) contained in the test solution 1, and the ordinate shows the nerve cell inducing ratio (%) 14 days after commencement of the culturing.

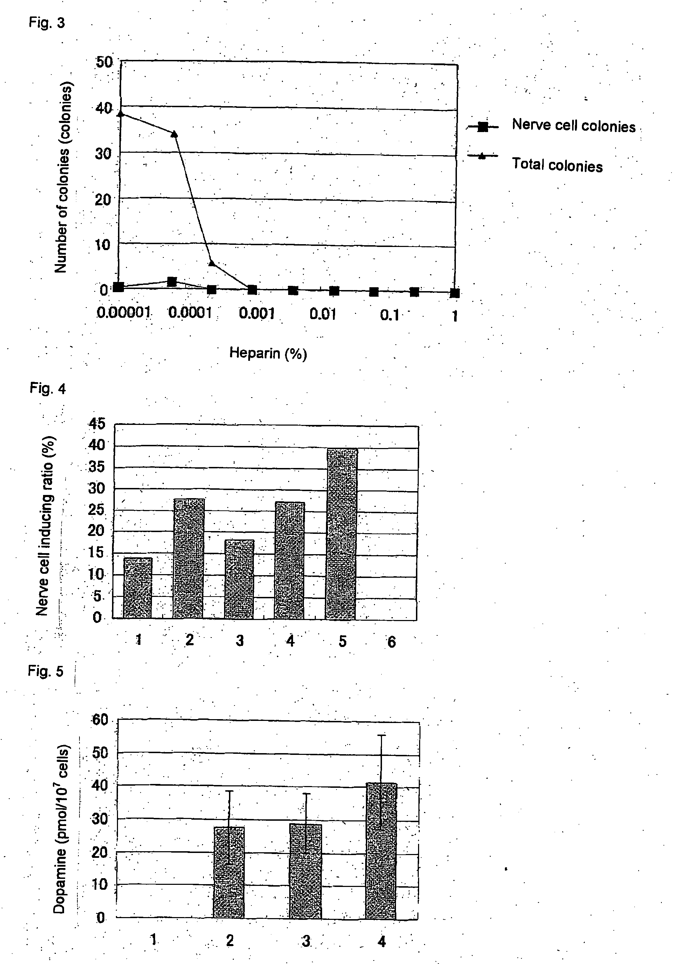

Fig. 3 is a graph showing the number of total colonies and the number of nerve cell colonies formed from EB5, when the concentration of heparin was changed under vitamin $B_{12}$ free conditions. In the graph, ■ shows nerve cell colonies, and ▲ shows total colonies. Also, in the graph, the abscissa shows the concentration of heparin (%) contained in the test solution 1, and the ordinate shows the number of colonies observed 14 days after commencement of the culturing.

Fig. 4 is a graph showing the ratio of nerve cells induced from EB5, namely nerve cell inducing ratio, when test solutions 2 to 7 were added instead of the test solution 1 in Example 1. In the graph, column 1 shows a test solution 2 addition condition, column 2 shows a test solution 3 addition condition, column 3 shows a test solution 4 addition condition, column 4 shows a test solution 5 addition condition, column 5 shows a test solution 6 addition condition and column 6 shows a test solution 7 addition condition. Also, in the graph, the ordinate shows the nerve cell inducing ratio (%) 14 days after commencement of the culturing.

Fig. 5 is a graph showing production of dopamine by nerve cells obtained by inducing differentiation of EB5. In the graph, column 1 shows PBS addition condition, column 2 shows a test solution 8 addition condition, column 3 shows a test solution 9 addition condition and column 4 shows a test solution 10 addition condition. Also, in the graph, the ordinate shows dopamine production (pmol/$10^7$ cells).per $10^7$ cells in which differentiation was induced, wherein the value is shown by mean value $\pm$ standard deviation (n = 3).

BEST MODE FOR CARRYING OUT THE INVENTION

1. Embryonic stem cell

In the present invention, the embryonic stem cell is a cell which can be cultured *in vitro* and can also be differentiated into all cells including reproductive cells when injected into the vascuole of an embryo of other individual before implantation, such as a blastocyst, and examples include the embryonic stem cells shown in the following (1), (2) and (3).

(1) It is known that embryonic stem cells of animal or the like established by culturing an initial embryo before implantation, more specifically, an ES cell established from an initial embryo-constituting inner cell mass, an EG cell (embryonic germ cell) established from a primordial germ cell, or a cell isolated from a cell group (e.g., primitive ectoderm) having a pluripotency of an early embryo before implantation, or a cell obtained by culturing such a cell, and an embryonal carcinoma cell established from teratocarcinoma (hereinafter referred also to as "EC cell"), also show the same property of the ES cell, so that they are included in the embryonic stem cell.

(2) An embryonic stem cell established by culturing an initial embryo prepared by carrying out nuclear transplantation of the nucleus of a somatic cell.

(3) An embryonic stem cell in which a gene on the chromosome of the embryonic stem cell of (1) or (2) is modified using a genetic engineering technique.

2. Methods for preparing embryonic stem cells

Methods for preparing the embryonic stem cells of the above-described (1), (2) and (3) are specifically described.

(1) Preparation of an embryonic stem cell established by culturing an early embryo of before implantation

By culturing an early embryo before implantation in accordance with the method described in a reference *(Manipulating the Mouse Embryo - A Laboratory Manual),* an embryonic stem cell can be prepared from the early embryo.

The method for culturing the thus obtained embryonic stem cell includes the methods for culturing embryonic stem cells described in references (Manipulating the Mouse Embryo - A Laboratory Manual; Methods in Enzymology, volume 225, Guide to Techniques in Mouse Development, Academy Press, 1993; *Preparation of Mutant Mice using ES cells)* and the like. It is possible to carry out serum-free culturing of the embryonic stem cell, and it can be sub-cultured while keeping its character as an undifferentiated embryonic stem cell, for example, using a medium prepared by supplementing Dulbecco's MEM medium with 15 to 20% of KNOCKOUT™ SR (manufactured by Life Technologies), 2 mmol/l glutamine, 100 μmol/l MEM NonEssential Amino Acids Solution, 50 U/ml penicillin, 50 U/ml streptomycin, 100 μmol/l mercaptoethanol and 1,000 U/ml LIF [Focus, 20, 8, (1998)].

(2) Preparation of an embryonic stem cell prepared by carrying out nuclear transplantation of the nucleus of a somatic cell

An egg into which the nucleus of a somatic cell of a mammal cell is transplated and in which normal development is started can be prepared, for example, in the following manner, using the methods described in Nature, 385, 810, (1997); Science, 280, 1256, (1998); Tanpaku Kakusan Koso (Protein, Nucleic Acid, Enzyme), 44, 892, (1999); Nature Biotechnology, 17, 456, (1999); Nature, 394, 369, (1998); Nature Genetics, 22, 127, (1999); Proc. Natl. Acad Sci. USA, 96, 14984, (1999); Nature Genetics, 24, 109, (2000) and the like.

An egg having the nucleus of other somatic cell and in which started normal development is started can be obtained by extracting the nucleus of a mammal cell and then initializing it (an operation for returning the nucleus to such a state that it can again repeat its development), allowing it to start its development using a method for injecting it into an enucleation-treated mammal unfertilized egg, and then culturing the egg which started its development.

As the method for initializing the nucleus of a somatic cell, plural numbers of methods are known. For example, the following method is known.

The initialization can be carried out by inducing the cell cycle into an interphase state ($G_0$ phase or $G_1$ phase) by changing the medium for culturing a nucleus donor side cell, from a medium containing 5 to 30%, preferably 10%, of fetal bovine serum (e.g., M2 medium) to an oligotrophic medium containing 0 to 1%, preferably 0.5%, of fetal bovine serum, and culturing for 3 to 10 days, preferably 5 days. This method is suitable when the mammal is, for example, sheep, goat, cattle or the like. In addition, the initialization can also be carried out by injecting the nucleus of the nucleus donor side cell into an enucleation-treated unfertilized egg of a mammal of the same species, and culturing it for several hours, preferably about 1 to 6 hours. This method is suitable when the mammal is, for example, mouse or the like.

The initialized nucleus can start its development in an enucleation-treated unfertilized egg. As the method for developing the initialized nucleus in the enucleation-treated unfertilized egg, plural numbers of methods are known. For example, development of an egg can be started through its activation by inducing the cell cycle into an interphase state ($G_0$ phase or $G_1$ phase) and transplanting the thus initialized nucleus into an enucleation-treated unfertilized egg of the same mammal species using an electroporation or the like method. This method is suitable when the mammal is, for example, sheep, goat, cattle or the like.

In addition, the development can be started by injecting a nucleus into an enucleation-treated unfertilized egg of the same mammal species, again transplanting the thus initialized nucleus into an enucleation-treated unfertilized egg of the same mammal species using a micromanipulator-aided method or the like, stimulating this with an egg activating substance (e.g., strontium or the like) and then inhibiting release of the second polar body through its treatment with a cell division inhibitor (e.g., cytochalasin B). This method is suitable when the mammal is, for example, mouse or the like.

Once an egg which started its development is obtained, an embryonic stem cell can be obtained using a conventionally known method described in *Manipulating The Mouse Embryo - A Laboratory Manual, Gene Targeting, Preparation of Mutant Mice Using ES cell* or the like.

(3) Preparation of an embryonic stem cell in which a gene on the chromosome is modified

An embryonic stem cell in which a gene on the chromosome is modified can be prepared using homologous recombination technique.

For example, as the chromosomal gene to be modified, a histocompatibility antigen gene, a gene concerned in a disease based on a disorder of a nervous system cell and the like can be cited.

Modification of the target gene on the chromosome can be carried out using a method described in *Manipulating The Mouse Embryo - A Laboratory Manual, Gene Targeting, Preparation of Mutant Mice Using ES cell* or the like.

Specifically, for example, a genomic gene of the target gene to be modified (e.g., a histocompatibility antigen gene, a disease-related gene or the like) is isolated, and a target vector for carrying out homologous recombination of the target gene is prepared using the isolated genomic gene. The embryonic stem cell in which a gene on the chromosome is modified can be prepared by introducing the thus prepared target vector into an embryonic stem cell and selecting a cell in which homologous recombination is initiated between the target gene and target vector.

The method for isolating a genomic gene of the target gene includes conventionally known methods described in Molecular Cloning, A Laboratory Manual, Second Edition, Cold Spring Harbor Laboratory Press (1989) (hereinafter referred to as "Molecular Cloning, Second Edition"), Current Protocols in Molecular Biology, John Wiley & Sons (1987 - 1997) (hereinafter referred to as "Current Protocols in Molecular Biology") and the like. In addition, a genomic gene of the target gene can be isolated by using a genomic DNA library screening system (manufactured by Genome Systems), Universal Genome Walker™ Kits (manufactured by CLONTECH) or the like.

The target gene for carrying out homologous recombination of the target gene can be prepared in accordance with the method described in *Gene Targeting, Preparation of Mutant Mice Using ES cell* or the like. The target vector can be used as either a replacement type or an insertion type.

As the method for efficiently selecting homologous recombinants, for example, methods such as the positive selection, promoter selection, negative selection and poly A selection described in *Gene Targeting, Preparation of Mutant Mice Using ES cell* and the like can be used. The methods for selecting a homologous recombinant of interest from the selected cell strains include a Southern hybridization method for genomic DNA *(Molecular Cloning,* Second Edition), a PCR method [PCR Protocols, Academic Press (1990)] and the like.

3. Nerve cell

In the present invention, the nerve cell which can be produced by inducing differentiation of an embryonic stem cell means a cell having a function to transmit a stimulus to other nerve cell or a muscle or glandular cell by receiving the stimulus from other nerve cell or a stimulus receptor cell.

Nerve cells are classified based on the difference of neurotransmitter produced by nerve cells, and specifically, they are classified based on the difference of neurotransmitter, neurotransmitter synthase and the like. The neurotransmitter includes both of the peptide types and non-peptide types. The non-peptide type neurotransmitter includes dopamine, noradrenaline, adrenaline, serotonin, acetylcholine, γ-aminobutyric acid and glutamic acid. The 3 kinds including dopamine, noradrenaline and adrenaline are generally called catecholamine.

The nerve cells classified based on these neurotransmitters includes, for example, dopaminergic neuron, acetylcholinergic neuron, γ-aminobutyratergic neuron, serotoninergic neuron, noradrenalinergic neuron, adrenalinergic neuron, glutamatergic neuron and the like. Dopaminergic neuron, noradrenalinergic neuron and adrenalinergic neuron are generally referred to as catecholaminergic neuron.

The catecholaminergic neurons express tyrosine hydroxylase in common, and noradrenalinergic neuron and adrenalinergic neuron express dopamine-β-hydroxylase in common. In addition, noradrenalinergic neuron, serotoninergic neuron, acetylcholinergic neuron and γ-aminobutyratergic neuron specifically express phenylethanolamic N-methyltransferase, tryptophan hydroxylase, choline acetyltransferase and glutamate decarboxylase, respectivelly. Accordingly, the methods for recognizing nerve cells includesuch as a discrimination method which uses an antibody capable of recognizing the above-described enzyme, and a method for detecting expression of mRNA coding for the above-described enzyme.

The peptide type neurotransmitter includes an adrenocorticotropic hormone [coticotropin (acth)], ααγ,β-lipotropin (lipotropin), α-melanocyte-stimulating hormone (msh), α-endorphin (endorphin), β-endorphin, γ-endorphin, methionine enkephalin (Met-enkephalin), leucine enkephalin (Leu-enkephalin), α-neoendorphin (neoendorphin), β-neoendorphin, dynorphin A, dynorphin B, leumorphin, vasopressin, neurophysin, oxytocin, neurophysin I, substance P, neurokinin A, neuropeptide K, neuropeptide-γ, neurokinin B, bombesin, gastrin-releasing peptide, secretin, motilin, glucagon, vasoactive intestinal peptide, growth hormone-releasing factor, insulin, insulin-like growth factors, somatostatin, gastrin, cholecystokinin, neuropeptide Y, pancreatic polypeptide, peptide YY, corticotropin-releasing factor, calcitonin, calcitonin gene-related peptide, angiotensin, bradykinin, thyrotropin-releasing hormone, neuterotensin, galanin, luteinizing hormone-releasing hormone and the like. The nerve cells which produce these peptide type neurotransmitters can be discriminated by detecting an antibody capable of recognizing a neurotransmitter or neurotransmitter precursor peptide or expression of mRNA encoding a neurotransmitter or neurotransmitter precursor peptide.

In addition, since nerve cells transmit information to skeletal muscle by secreting acetylcholine from its nerve ending, they are classified into acetylcholinergic neuron. The marker protein of motor neuron includes islet 1 [Nature, 344, 879, (1990)] and the like.

The production process of the present invention is used for the production of a nerve cell, preferably a catecholaminergic neuron, more preferably a dopaminergic neuron.

Particularly, the dopaminergic neuron produced by the method of the present invention through its differentiation induction from an embryonic stem cell expresses tyrosine hydroxylase whose expression is commonly observed in catecholaminergic neurons as described above. It is characterized by a cell which does not express dopamine-$\beta$-hydroxylase whose expression is commonly observed in noradrenalinergic neuron and adrenalinergic neuron, and can improve symptoms of neurodegenerative diseases such as Parkinson's disease by its transplantation.

4. Heparin and substance having heparin-like activity

Heparin is a group of sulfated (sulfonated) mucopolysaccharides, and these are also called glycosaminoglycan. Structure of heparin is characterized by a disaccharide unit comprising $\alpha$-1,4-glycoside-bound D-glucosamine and L-iduronic acid units and a disaccharide unit comprising $\alpha$-1,4-glycoside-bound D-glucosamine and D-glucuronic acid units. The position and number of sulfate group (sulfo group) are both variable. These may be bound via oxygen (O-sulfation) and nitrogen (N-sulfation). The iduronic acid residue is 2-O-sulfated in most cases, and the glucosamine residue is N-sulfated in most cases, but 6-O-sulfation is also carried out as occasion demands. The glucuronic acid residue is not sulfated in most cases. Next, the disaccharide units form a heparin molecule through their mutual $\alpha$-1,4-glycoside binding. The number and arrangement of these disaccharide units are also variable. Accordingly, heparin comprises many structurally different molecules, and they can be distinguished, for example, by an elemental analysis or based on their chain lengths, molecular weights or electric charges. In general, heparin means a mixture of structurally different heparin molecules of the above-described type ($\alpha$-heparin), and as occasion demands, it may contain other components such as $\beta$-heparin (also called chondroitin sulfate B or dermatan sulfate) and/or other cellular components (particularly protein). Heparin may be present as free acid or in the form of a physiologically acceptable salt. The physiologically acceptable salt of heparin includes sodium, calcium or magnesium salt and the like.

Molecular weight of the heparin molecule is generally within the range of 200 to 30,000 Da. The heparin used in the present invention may be a heparin having a molecular weight of within this range or a fragmentation product thereof. Among them, it is desirable to use a mixture of heparin molecules having weight average molecular weights of about 500 to about 10,000 Da (LMW heparin). The fragmentation can be carried out preferably by controlled partial cleavage of heparin in a chemical, enzymatic and physical manner. The chemical cleavage can be carried out, for example, using sodium nitrate, the enzymatic chemical cleavage can be carried out, for example, using a heparinase derived from *Flavobacterium,* and the physical chemical cleavage can be carried out, for example, by an ultrasonic wave.

The substance having heparin-like activity means a group of substances having the activity to inhibit blood coagulation and thrombus formation like heparin. Specific examples include a sulfated plant oligosaccharide or polysaccharide such as alginic acid, pectin, xylan or starch, or a polsulfuric acid ester prepared from dextran, and a sulfated animal glycosaminoglycan and the like. These substances having heparin-like activity may be obtained from natural materials or prepared by semi-synthesis or complete synthesis, and the synthesis is generally carried out by sulfation of the above-described plant or animal polysaccharide, for example, by allowing it to react with chlorosulfonic acid and then neutralizing the released hydrochloric acid with a base.

In the production process of the present invention, concentration of heparin, a substance having heparin-like activity or a salt thereof in the culture medium is preferably 0.00001 to 5% by weight, more preferably 0.0001 to 1% by weight, and most preferably 0.001 to 0.1% by weight, as heparin or the substance having heparin-like activity.

5. Vitamin $B_{12}$

The vitamin $B_{12}$ used in the present invention includes, for example, hydroxocobalamin hydrochloride, hydroxocobalamin acetate, hydroxocobalamin, cyanocobalamin, methylcobalamin, nitrosocobalamin, adenosylcobalamin, aquacobalamin and salts thereof and the like.

In the production process of the present invention, the concentration of vitamin $B_{12}$ or a salt thereof in the culture medium is preferably 100 pmol/l to 10 $\mu$mol/l, more preferably 1 nmol/l to 10 $\mu$mol/l, and most preferably 10 nmol/l to 10 $\mu$mol/l, as vitamin $B_{12}$.

6. Biotin

Biotin is a kind of vitamin B family and is also called vitamin H, and biotin and a salt thereof are used in the present invention. In the production process of the present invention, the concentration of biotin or a salt thereof in the culture medium is preferably 0.001 to 10 $\mu$g/ml, and more preferably 0.01 to 1 $\mu$g/ml, as biotin.

7. Polyamine

The polyamine used in the present invention includes a non-proteinous aliphatic amine having plural numbers of amino groups, and specific examples include biogenic amines such as putrescine, spermine, cadaverine and spermidine. In the production process of the present invention, the concentration of polyamine in the culture medium is preferably 0.001 to 10 $\mu$g/ml, and more preferably 0.01 to 1 $\mu$g/ml.

8. Iron-containing compound

The iron-containing compound to be used in the present invention includes a compound containing iron ion (II) or iron ion (III), and preferably, a compound containing iron ion (II) is used.
The compound containing iron ion (II) includes, for example, an anhydride or hydrate containing iron ion (II) such as $FeCl_2$, $FeSO_4$, $FeSO_4 \cdot (NH_4)_2SO_4$, $Fe[CH_3CH(OH)COO]_2$ or $K_4[Fe(CN)_6]$.
The compound containing iron ion (III) includes, for example, an anhydride or hydrate containing iron ion (III) such as $FeCl_3$, $Fe_2(SO_4)_3$, $Fe_2(SO_4)_3 \cdot (NH_4)_2SO_4$, $Fe(NO_3)_3$ or $K_3[Fe(CN)_6]$.
In the production process of the present invention, the concentration of the iron-containing compound in the culture medium is preferably 0.01 to 10 $\mu$mol/l, and more preferably 0.1 to 1 $\mu$mol/l, as iron ion (II) or iron ion (III).

9. Preparation of medium

The medium used in the production process of the present invention can be prepared using a medium which is used in culturing animal cells, as the basal medium.
As the basal medium, any one of the media which can be used in culturing animal cells can be used, such as BME medium [Proc. Soc. Exp. Biol. Med., 89, 362, (1965)], BGJb medium [Exp. Cell Res., 25, 41, (1961)], CMRL 1066 medium [N.Y. Academy of Sciences, 5, 303, (1957)], Glasgow MEM medium [Virology, 16, 147, (1962)], Improved MEM Zinc Option medium [J. National Cancer Inst., 49, 1705, (1972)], IMDM medium [In Vitro, 9, 6, (1970)], Medium 199 medium [Proc. Soc. Exp. Biol. Med., 73, 1, (1950)], Eagle MEM medium [Science, 130, 432, (1959)], Alpha MEM medium [Nature New Biology, 230, 310, (1971)], Dulbecco MEM medium [Virology, 8, 396, (1959)], Ham medium [Exp. Cell Res., 29, 515, (1963); Proc. Natl. Acad. Sci. USA, 53, 288, (1965)], RPMI 1640 medium [J. A. M A., 199, 519, (1967)], Fischer's medium [Methods in Med. Res., 10, (1964)], McCoy's medium [Proc. Soc. Exp. Biol. Med., 100, 115, (1959)], Williams E medium [Exp. Cell Res., 69, 106, (1971); Exp. Cell Res., 89, 139, (1974)] and a mixed medium thereof and the like.
Also, any one of the media for embryo culture described in Manipulating The Mouse Embryo - A Laboratory Manual, Methods in Enzymology, volume 225, Guide to Techniques in Mouse Development, Academic Press, (1993), Preparation of Mutant Mice Using Embryonic Stem Cell and the like, such as M2 medium, M16 medium, Whitten medium, medium for *in vitro* fertilization and the like, can be used as the basal medium so long as it is a medium which can be used in the culturing of embryo.
In addition, even in the case of a medium supplemented with various growth factors as substitutes for serum or a protein-free medium, any one of such media can be used so long as animal cells and embryos can be cultured. Specific examples include a serum-free medium supplemented with KNOCKOUT™ SR [M.D. Goldsborough et al.; Focus, 20, 8, (1998)], a serum-free medium supplemented with insulin and transferrin, a medium supplemented with a cell-derived factor and the like.
The serum-free medium supplemented with insulin and transferrin includes CHO-S-SFM II (manufactured by GIBCO BRL), Hybridoma-SFM (manufactured by GIBCO BRL), eRDF Dry Powdered Media (manufactured by GIBCO BRL), UltraCULTURE™ (manufactured by BioWhittaker), UltraCHODOMA™ (manufactured by BioWhittaker), Ultra-CHO™ (manufactured by BioWhittaker), UltraMDCK™ (manufactured by BioWhittaker), ITPSG medium [S. Hosoi et al.; Cytotechnology, 5, S17, (1991)], ITSFn medium [A. Rizzino and C. Growley; Proc. Natl. Acad Sci. USA, 77, 457, (1980)], mN3 medium [S. Okabe et al.; Mech. Dev., 59, 89, (1996)] and the like.
The medium supplemented with a cell-derived factor includes a medium supplemented with a culture supernatant of a multipotential teratocarcinoma cell PSA 1 (G.R. Martin; Proc. Natl. Acad Sci. USA, 78, 7634, 1981).
The protein-free medium includes CD-CHO (manufactured by GIBCO BRL), PFHM-II (manufactured by GIBCO BRL), UltraDOMA-PF™ (manufactured by BioWhittaker) and the like.
A medium for the differentiation induction of embryonic stem cell can be prepared by adding the above-described vitamin $B_{12}$ or a salt thereof and heparin, a substance having heparin-like activity or a salt thereof to such a medium. For example, it can be prepared by adding 10% of KNOCKOUT™ SR (manufactured by GIBCO BRL), 2 mmol/l glutamine, 50 U/ml penicillin, 50 U/ml streptomycin and 0.1 mmol/l 2-mercaptoethanol, and vitamin $B_{12}$ or a salt thereof and heparin, a substance having heparin-like activity or a salt thereof, to Dulbecco's MEM medium. In addition, it is desirable to add the above-described one or more compounds selected from biotin or a salt thereof, a polyamine and an iron-containing compound to this medium.

10. Culture vessel

As the culture vessel to be used in the present invention, any culture vessel can be used so long as it can culture embryonic stem cells, but preferably, a culture vessel which is used for cell culture is desirable. The culture vessel for cell culture includes, for example, flask, flask for bacterium culture, flask for cell culture, dish, Petri dish, dish for tissue culture, Conzer dish, Parmanox dish, multi-dish, microplate, micro-well plate, multi-plate, multi-well plate, separate strip well, Terasaki plate, chamber slide for tissue culture, Petri Schale, Petri Schale for cell culture, tube for tissue culture, tray, tray for cell culture, Cellfactory, culture bag, Technopot, roller bottle, spinner, hollow fiber and the like. In order to control adhesiveness of cells to the culture vessel, an artificial treatment can also be applied to the surface of the cell-contacting side of the culture vessel. Examples of the artificial treatment of the surface of culture vessel include collagen type I coat, collagen type IV coat, gelatin coat, poly-L-lysine coat, fibronectin coat, laminin coat, proteoglycan coat, glycoprotein coat, matrigel coat, silicon coat and the like, and collagen type I coat, collagen type IV coat, gelatin coat, fibronectin coat and laminin coat are suitably used. In addition, it can also be treated in such a manner that it has negative charge, like the case ofPrimaria (manufactured by FALCON). Culture vessels to which such treatments were applied can also be used in the production process of the present invention.

11. Process for producing nerve cell by inducing differentiation of embryonic stem cell

Specifically, the production process of the present invention includes a step culturing an embryonic stem cell under non-aggregation conditions using vitamin $B_{12}$ or a salt thereof and heparin, a substance having heparin-like activity or a salt thereof to thereby induce differentiation into a nerve cell, and isolating a nerve cell from the culture.

In the production process of the present invention, the differentiation induction can be carried out without utilizing a specific proteinous factor such as a proteinous factor having the activity owned by a stroma cell to induce differentiation of an embryonic stem cell into an ectodermal cell or an ectoderm-derived cell (hereinafter referred to as "SDIA activity"). The specific proteinous factor such as a proteinous factor having the SDIA activity includes a secreted frizzled-related protein (hereinafter referred to as "SFRP") 1, a polypeptide in which 1 or more amino acids constituting SFRP 1 are deleted, substituted or added and which has the activity to induce differentiation of an embryonic stem cell into an ectodermal cell or an ectoderm-derived cell, and the like (WO03/42384).

The culturing of an embryonic stem cell under non-aggregation conditions means that its culturing is started under a single cell state by disengaging mutual adhesion of cells, followed by culturing continuously. Also, the state of single cell means a state under which the cells are separated from one another without their mutual adhesion by an enzyme digestion or the like.

In the production process of the present invention, the inoculated cells do not aggregate and do not form an embryoid body. In order to start culturing of an embryonic stem cell under such a single cell state and continue the culturing, it is desirable to start the culturing by inoculating at a cell density lower than the cell density generally used in culturing an embryonic stem cell for the sub-culturing of the embryonic stem cell. Specifically, treatment such as an enzyme digestion is applied to embryonic stem cells and a cell suspension of a single cell state is prepared using a medium, and the cell suspension is cultured in such a manner that the cells can be present in the culturing system under such a state that they do not contact with each other. Such a culturing is different from the culturing method which uses the embryo body for the purpose of inducing differentiation by positively effecting aggregation of the cells and thereby reproducing a false embryo state. In this case, regarding the cell density of embryonic stem cells for inoculation use at such a degree that the cells can be present in the culturing system under such a state that they do not contact with each other, it is preferably from scores to thousands of cells/cm$^2$, more preferably 30 to 300 cells/cm$^2$.

The method for obtaining embryonic stem cells of a single cell state includes a conventionally known enzyme digestion method which is used in tissue and cell cultures. Specifically, the medium is removed from a culture dish containing cultured embryonic stem cells grown to several ten percent to almost confluent state by carrying out medium exchange on the preceding day, and then the cells are washed several times, preferably 2 to 3 times, using phosphate buffered saline (to be referred to as "PBS" hereinafter). After the washing, an appropriate enzyme digestion liquid (e.g., PBS containing 1 mmol/l EDTA and 0.25% trypsin) is added to the culture dish containing embryonic stem cells which are then cultured at 37°C for scores of minutes, preferably 5 to 20 minutes. After the enzyme reaction, the cells are suspended in a medium which is used for inducing differentiation of embryonic stem cells and subjected to centrifugation (e.g., 5 minutes at 4°C, 200 × g), and the thus obtained cells are again suspended in the medium to recover the embryonic stem cells under a state of single cells.

The differentiation of the embryonic stem cells prepared into a single cell state into nerve cells can be induced by a culture method suitable for inducing differentiation of embryonic stem cells, specifically, monolayer culture method, microcarrier method, perfusion culture method, soft agar culture method or the like, using the medium described in the above-described 9 and the culture vessel described in the above-described 10. In order to induce differentiation of the embryonic stem cell into a nerve cell, it is desirable to effect it by continuing the culturing using a production process including the above-described process, while optionally carrying out medium exchange.

The differentiation of the embryonic stem cells into nerve cells is induced by the production process of the present invention, and 5% or more, preferably 15% or more, more preferably 40% or more, of the embryonic stem cells subjected to the production process of the present invention can be differentiation-induced into nerve cells.

12. Application of nerve cells produced by the production process of the present invention

The nerve cells produced by the production process of the present invention can be applied, for example, to the treatment of diseases based on the cell injury of nerve cells.

The diseases based on the injury of nerve cells include, for example, Alzheimer disease, Huntington chorea, Parkinson disease, ischemic cerebral disease, epilepsy, brain injury, spinal injury, motor neuron disorder, neurodegenerative disease, retinitis pigmentosa, inner ear deafness, Down syndrome, multiple sclerosis, amyotrophic lateral sclerosis, diseases caused by the neurotoxin obstruction and the like.

For the treatment of diseases based on cytotoxicity, a cell having the same function of the damaged cell, a precursor cell of the damaged cell, a cell capable of compensating function of the damaged cell, a cell having a function to accelerate regeneration of the damaged cell and the like, which can be applied to the transplantation medical treatment, are used.

When it is used for the purpose of transplantation medical treatment, it is preferable to use a nerve cell produced by the production process of the present invention by purifying it.

The purification method of cells includes any conventional method for separating and purifying cells, and specifically, the methods using flow cytometry described in Antibodies, A Laboratory Manual, Cold Spring Harbor Laboratory, Chapter 14 (1988); Monoclonal Antibodies: Principles and Practice, Third Edition, Acad. Press (1993); Antibody Engineering, A Practical Approach, IRL Press at Oxford University Press (1996); Int. Immunol., 10, 275, (1998), Exp. Hematol., 25, 972, (1997) and the like, the panning methods described in Monoclonal Antibodies, Antibody Engineering, J. Immunol., 141, 2797, (1988) and the like, the cell fractionation methods which use density difference of sucrose concentration, described in Soshiki Baiyo No Gijutsu (Techniques of Tissue Culture) (Third Edition), Asakura Shoten (1996) and the like, and so on.

The method for improving purity of the nerve cell produced by the present invention includes a step of culturing the embryonic stem cell under non-aggregation conditions using vitamin $B_{12}$ or a salt thereof and heparin, a substance having heparin-like activity or a salt thereof, and then culturing in a medium containing an anticancer agent, as described above. By this step, cells of undifferentiated state can be removed, and nerve cells having higher purity can therefore be obtained. That is, cells other than the nerve cell of interest, such as undifferentiated cells, can be removed by treating with an anticancer agent.

The anticancer agent used in the method for improving purity of the nerve cell produced by the present invention includes mitomycin C, 5-fluorouracil, adriamycin, Ara-C, methotrexate and the like. It is preferable to use these anticancer agents at a concentration which shows higher cytotoxicity upon cells of undifferentiated state than the nerve cells after differentiation induction. For example, a concentration of 1/100 to 1 of the concentration described in *The Pharmacopoeia of Japan,* in the case of using these anticancer agents in the living body, is desirable.

The step for culturing the nerve cell obtained by inducing differentiation of an embryonic stem cell in a medium containing an anticancer agent includes, for example, a method in which an appropriate concentration of anticancer agent, such as mitomycin C at 1 to 100 $\mu$g/ml, preferably 10 $\mu$g/ml, is added to a medium-exchanged culture system on the preceding day, and the cells are cultured at 37°C for several hours, preferably 2 to 3 hours, in a $CO_2$ incubator ventilated with 5% of carbon dioxide.

As the medium used in this case, any medium which can carry out culturing of cells after inducing differentiation can be used. Specific examples include the medium described in the above-described 9 and the like.

In the transplantation medical treatment, the rejection due to the difference of histocompatibility antigens often causes problems, but the rejection due to the difference of histocompatibility antigens can be avoided by the use of the embryonic stem cell in which the nucleus of a somatic cell was treated by nuclear transplantation described in the above-described 2(2), or the embryonic stem cell in which a gene on the chromosome is modified, described in the above-described 2(3).

Also, by inducing differentiation of the embryonic stem cell in which the nucleus of a somatic cell was treated by nuclear transplantation described in the above 2(3), a nerve cell of the individual who provided the somatic cell can be obtained. Such a cell of individual is effective by the cell itself in the transplantation medical treatment, and is also effective as a diagnostic material for judging whether or not an existing drug is effective for the individual. In addition, it is possible to judge sensitivity for oxidation stress and aging by culturing the cells after inducing differentiation for a long period of time, and the risk of the individual for diseases such as neurodegenerative disease can be evaluated by comparing with cells of other individuals in terms of their functions and life spans, and such evaluation data are useful in providing an effective method for preventing a disease diagnosed as a high morbidity rate in the future.

As the transplantation method, any method can be used, so long as it is a method suitable for the disease to be treated, and conventionally known methods suitable for respective diseases are known. For example, a disease can be treated by preparing the embryonic stem cell of the above-described 2(2) or(3) from a somatic cell of a disease patient, inducing differentiation of the embryonic stem cell into a nerve cell, and then transplanting it to the affected part of the patient. Specifically, the methods described in Nature Neuroscience, 2, 1137, (1999), and the like can be cited as a method for transplanting a nerve cell to patients of Parkinson disease.

Examples of the present invention are shown in the following.

Example 1

Differentiation induction of ES cell into nerve cell by heparin and vitamin B 12 (1)

In the following Examples, an ES cell EB5 [Nature Genet., 24, 372, (2000)] was used as the embryonic stem cell.

The EB5 was inoculated into a dish coated with 0.1% of gelatin and sub-cultured at 37°C in a $CO_2$ incubator ventilated with 5% of carbon dioxide while keeping its undifferentiated state, using G-MEM (Invitrogen) containing 1% fetal bovine serum (manufactured by JRH Biosciences), 10% Knockout Serum Replacement (manufactured by Invitrogen), 1 mmol/l pyruvic acid (manufactured by Sigma), 0.1 mmol/l nonessential amino acids (manufactured by Invitrogen), 0.1 mmol/l 2-mercaptoethanol (manufactured by Nacalai Tesque), 20 mg/ml blasticidin (manufactured by Invitrogen) and $2 \times 10^3$ U/ml ESGRO (manufactured by Chemicon) (to be referred to as "maintenance medium" hereinafter).

When differentiation of EB5 is induced, $2.5 \times 10^3$ cells of EB5 were inoculated into a 24 well plate coated with 0.1% of gelatin, a medium prepared by eliminating serum, blasticidin and ESGRO from the meitenance medium (hereinafter referred to as "differentiation induction medium") was added at 1 ml to respective wells, and the cells were allowed to adhere thereto by incubating at 37°C for 2 hours. Thereafter, 500 $\mu$l of PBS containing various concentration of heparin (manufactured by Sigma) or vitamin $B_{12}$ (cyanocobalamin, manufactured by Nacalai Tesque) (hereinafter referred to as "test solution 1") was added thereto and incubated at 37°C for 14 days without changing the medium. Each test condition was carried out in tandem. In this connection, the concentrations of heparin and vitamin $B_{12}$ mean their concentrations in the test solution 1.

On the 14th day after addition of the test solution 1, colonies having a diameter of 50 $\mu$m or more were counted using an optical microscope to calculate the number of total colonies. Also, among the colonies, a colony on which neurite was observed was regarded as a nerve cell colony, and the nerve cell inducing ratio was calculated by the following formula.

$$\text{Nerve cell inducing ratio (\%)}$$

$$= \text{the number of nerve cell colonies/the number of total colonies} \times 100$$

As a result, when vitamin $B_{12}$ was added, difference in the number of total colonies was not found between the heparin 0% addition condition and 0.01% addition condition, independent of the concentration of vitamin $B_{12}$ (Fig. 1). On the other hand, it was found that nerve cells are hardly induced under the heparin 0% addition condition even when vitamin $B_{12}$ is added, while nerve cells are induced under the heparin 0.01% addition condition depending on the concentration of vitamin $B_{12}$ (Fig. 2).

In addition, when vitamin $B_{12}$ was not added, nerve cells were hardly induced independent of the heparin concentration (Fig. 3).

Based on the above results, it was found that differentiation of embryonic stem cells into nerve cells is induced by a combination of heparin and vitamin $B_{12}$.

Example 2

Differentiation induction of ES cell into nerve cell by heparin and vitamin B12 (2)

Nerve cell inducing differentiation ratio was examined in the same manner as in Example 1, except that PBS containing 0.01% of heparin and 100 nmol/l of vitamin $B_{12}$ (hereinafter referred to as "test solution 2"), a test solution 3 containing 0.1 $\mu$g/ml of biotin [(+)-biotin, manufactured by Wako Pure Chemical Industries] in the test solution 2, a test solution 4 containing 0.42 $\mu$g/ml of $K_4[Fe(CN)_6]$ (manufactured by Nacalai Tesque) in the test solution 2, a test solution 5 containing 0.081 µg/ml of putrescine (manufactured by Sigma) in the test solution 2, a test solution 6 containing 0.1 µg/ml of biotin, 0.42 µg/ml of $K_4[Fe(CN)_6]$ and 0.081 µg/ml of putrescine in the test solution 2 and a test solution 7 containing 0.01% of heparin, 0.1 mg/ml of biotin, 0.42 µg/ml of $K_4[Fe(CN)_6]$ and 0.081 µg/ml of putrescine were used instead of the test solution 1 in the above-described Example 1.

As a result, in comparison with the addition condition of the test solution 2 (column 1), the nerve cell differentiation induction ratio was increased in all of the addition condition of the test solution 3 (column 2), addition condition of the test solution 4 (column 3), addition condition of the test solution 5 (column 4) and addition condition of the test solution 6 (column 5), and particularly, the nerve cell differentiation induction ratio was increased most significantly in the addition condition of the test solution 6 (Fig. 4). In this connection, nerve cells were not induced under the addition condition of the test solution 7 (column 6) (Fig. 4).

Based on the above results, it was found that the activity to induce differentiation of embryonic stem cells into nerve cells by the combination of heparin and vitamin $B_{12}$ is reinforced by the addition of biotin, putrescine or an iron-containing compound.

In addition, in order to examine properties of the nerve cells obtained by inducing differentiationn, immunostaining of nerve cell colonies with various antibodies for NCAM (neural cell adhesion molecule) as a nerve tissue marker, class III β-tubulin as a mature nerve cell marker and tyrosine hydroxylase (TH) as a catecholaminergic neuron marker was carried out in the following manner.

After inducing differentiation into nerve cells, the medium was removed from the 24 well plate, and the cells in each well were washed once with 1 ml of PBS and then treated with 0.25 ml of PBS containing 4% (w/v) paraformaldehyde at room temperature for 15 minutes. Thereafter, they were washed twice with 0.5 ml of PBS and then treated at -20°C for 15 minutes after adding 0.25 ml of methanol. Subsequently, the cells were washed twice with 0.5 ml of PBS and then subjected to 2 hours of blocking at room temperature after adding 0.25 ml of PBS containing 2% (w/v) skim milk (manufactured by Difco). As the primary antibodies, a rabbit-derived anti-NCAM antibody (manufactured by Chemicon) was diluted 1/400 times, and a mouse-derived anti-βIII-tubulin antibody (TuJ 1, manufactured by Covance) 1/400 times and a rabbit-derived anti-TH antibody (manufactured by Chemicon) 1/200 times, with PBS containing 2% (w/v) skim milk, and incubated overnight at 4°C. After the primary antibody reaction and subsequent 3 times of washing with 0.25 ml of PBS containing 0.05% (w/v) Tween 20, the reaction was carried out at room temperature for 1 hour using an anti-mouse IgG-HRP (manufactured by DAKO, 1/100 times dilution) or an anti-rabbit IgG-HRP (manufactured by DAKO, 1/100) as the secondary antibody. They were washed 3 times with 0.25 ml of PBS containing 0.05% (w/v) Tween 20 and then allowed to develop color using TrueBlue (manufactured by KPL).

As a result, all of the nerve cell colonies were positive regarding NCAM, βIII-tubulin and TH, thus revealing that the catecholaminergic neuron was induced.

Example 3

Differentiation induction of ES cell into nerve cell by heparin and vitamin B12 (3)

Differentiation induction of EB5 was carried out in the same manner as in Example 1, except that 8 well plate coated with 0.005% of poly-L-ornithine (manufactured by SIGMA) and 50 µg/ml of fibronectin (manufactured by Invitrogen) was used instead of the 24 well plate which was coated with 0.1% of gelatin and used in Example 1, and PBS, PBS containing 0.01% of heparin and 10 nmol/l of vitamin $B_{12}$ (to be referred to as "test solution 8" hereinafter), PBS containing 0.01% of heparin and 1000 nmol/l of vitamin $B_{12}$ (to be referred to as "test solution 9" hereinafter) and PBS containing 0.01% of heparin, 10 nmol/l of vitamin $B_{12}$, 0.3 µg/ml of biotin, 3 µmol/l of $K_4[Fe(CN)_6]$ (manufactured by Nacalai Tesque) and 1.6 µg/ml of putrescine (to be referred to as "test solution 10" hereinafter) were used instead of the test solution 1.

Total RNA was recovered from the cells 14 days after commencement of the differentiation induction using SV Total RNA Isolation System (manufactured by Promega). From the thus recovered total RNA, cDNA was prepared using Superscript™ II RNase H Reverse Transcriptase (manufactured by Invitrogen) and oligo(dT)$_{12-18}$ primer (manufactured by Invitrogen). DNA fragments having the nucleotide sequences represented by SEQ ID NO:1 and SEQ ID NO:2 were used as the primers for detecting the transcription product of *Nurr*1 gene as a marker for the midbrain dopaminergic neuron, and DNA fragments having the nucleotide sequences represented by SEQ ID NO:3 and SEQ ID NO:4 were used as the primers for detecting the transcription product of *TH* gene. In addition, as a control, DNA fragments having the nucleotide sequences represented by SEQ ID NO:5 and SEQ ID NO:6 were used as the primers for detecting the transcription product of glyceraldehyde 3-phosphate dehydrogenase (GAPDH) gene. GeneAmp PCR system 9700Ex (manufactured by Applied Biosystems) was used in the PCR reaction, and 30 cycles of the reaction of 94°C for 30 seconds, 60°C for 30 seconds and 72°C for 30 seconds were carried out using Ex Taq (manufactured by Takara) in each case. The reaction solutions were analyzed by agarose gel electrophoresis and ethidium bromide staining.

As a result, the *Nurr*1 and TH genes were detected in each addition condition of the test solutions 8, 9 and 10, so that it was found that the dopaminergic neuron was induced. In this connection, the *Nurr*1 and *TH* genes were not detected under the PBS addition condition.

In addition, in order to measure dopamine production by the nerve cells obtained by inducing differentiation under the conditions of this test, the cells were subjected to the following test.

The cells 14 days after commencement of the induction were washed 3 times with PBS and incubated at 37°C for 24 hours by adding G-MEM medium (manufactured by Invitrogen). The supernatant was recovered, and EDTA in final concentration of 0.1 mmol/l and perchlonic acid in final concentration of 0.1 mol/l were added thereto. After carrying out centrifugation at 4°C and at 15000 rpm for 15 minutes, the supernatant was passed through a 0.22 mm filter (manufactured by Millipore, SLGV013NL) to prepare measuring samples. Using TSK-GEL SUPERODS (manufactured by Tosoh) column at a column oven setting temperature of 40°C, and using a 100 mmol/l citrate buffer:methanol (97:3) solution, to which 5 mmol/l octane sulfonate sodium and 0.1 mmol/l EDTA·2Na were added, as the eluent, the measuring samples were analyzed at a flow rate of 1.2 ml/min. An electrochemical detector (manufactured by Tosoh, EC8020) was used for the detection. As a standard sample, a commercially available dopamine (manufactured by Nacalai Tesque, Cat. 14212-71) was analyzed in the same manner, and dopamine was determined based on the peak area ratio obtained by analyzing each sample.

As a result, dopamine was detected in each addition condition of the test solutions 8 (column 2), 9 (column 3) and 10 (column 4), so that it was found that the dopaminergic neuron was induced (Fig. 5). In this connection, dopamine was not detected under the PBS addition condition (column 1).

INDUSTRIAL APPLICABILITY

The present invention provides a process for producing a nerve cell by inducing differentiation of an embryonic stem cell, a method for inducing differentiation of the embryonic stem cell into a nerve cell, a medium to be used in the production process or differentiation induction method, or a method for improving purity of the nerve cell obtained by inducing differentiation of the embryonic stem cell.

Free Text of Sequence Listings

SEQ ID NO:1 - Description of artificial sequence: Synthetic DNA
SEQ ID NO:2 - Description of artificial sequence: Synthetic DNA
SEQ ID NO:3 - Description of artificial sequence: Synthetic DNA
SEQ ID NO:4 - Description of artificial sequence: Synthetic DNA
SEQ ID NO:5 - Description of artificial sequence: Synthetic DNA
SEQ ID NO:6 - Description of artificial sequence: Synthetic DNA

**Claims**

1. A process for producing a nerve cell, which comprises a step of culturing an embryonic stem cell under non-aggregation conditions using vitamin $B_{12}$ or a salt thereof and heparin, a substance having heparin-like activity or a salt thereof to thereby induce differentiation into a nerve cell, and isolating a nerve cell from the culture.

2. The process according to claim 1, wherein the nerve cell is a catecholaminergic neuron.

3. The process according to claim 2, wherein the catecholaminergic neuron is a dopaminergic neuron.

4. The process according to any one of claims 1 to 3, wherein the differentiation induction is carried out in a serum-free medium.

5. The process according to claim 4, wherein the differentiation is induced without utilizing such activity owned by a stroma cell that induces differentiation of an embryonic stem cell into an ectodermal cell or an ectoderm-derived cell.

6. The process according to claim 4 or 5, wherein the serum-free medium is a serum-free medium which comprises one or more compounds selected from biotin or a salt thereof, a polyamine and an iron-containing compound.

7. A medium having activity of inducing differentiation of an embryonic stem cell into a nerve cell, which is used for the process described in any one of claims 1 to 6.

8. A method for inducing differentiation of an embryonic stem cell into a nerve cell, which comprises a step of culturing the embryonic stem cell under non-aggregation conditions using vitamin $B_{12}$ or a salt thereof and heparin, a substance having heparin-like activity or a salt thereof.

9. The method according to claim 8, wherein the nerve cell is a catecholaminergic neuron.

10. The method according to claim 9, wherein the catecholaminergic neuron is a dopaminergic neuron.

11. The method according to any one of claims 8 to 10, wherein the differentiation induction is carried out in a serum-free medium.

12. The method according to claim 11, wherein said differentiation is induced utilizing such activity owned by a stroma cell that induces differentiation of an embryonic stem cell into an ectodermal cell or an ectoderm-derived cell.

13. The method according to claim 11 or 12, wherein the serum-free medium is a serum-free medium which comprises one or more compounds selected from biotin or a salt thereof, a polyamine and an iron-containing compound.

14. A medium having activity of inducing differentiation of an embryonic stem cell into a nerve cell, which is used for the method described in any one of claims 8 to 13.

15. A method for improving purity of a nerve cell obtained by inducing differentiation of an embryonic stem cell, which comprises steps of culturing the embryonic stem cell under non-aggregation conditions using vitamin $B_{12}$ or a salt thereof and heparin, a substance having heparin-like activity or a salt thereof, and then culturing in a medium containing an anticancer agent.

16. The method according to claim 15, wherein the anticancer agent is an anticancer agent selected from the group consisting of mitomycin C, 5-fluorouracil, adriamycin, Ara-C and methotrexate.

Fig. 1

Fig. 2

Fig. 3

Fig. 4

Fig. 5

## INTERNATIONAL SEARCH REPORT

| International application No. |
| --- |
| PCT/JP2005/012472 |

A. CLASSIFICATION OF SUBJECT MATTER
   Int.Cl⁷ C12N5/06

According to International Patent Classification (IPC) or to both national classification and IPC

B. FIELDS SEARCHED

Minimum documentation searched (classification system followed by classification symbols)
   Int.Cl⁷ C12N5/06

Documentation searched other than minimum documentation to the extent that such documents are included in the fields searched
   Jitsuyo Shinan Koho          1922-1996   Jitsuyo Shinan Toroku Koho   1996-2005
   Kokai Jitsuyo Shinan Koho    1971-2005   Toroku Jitsuyo Shinan Koho   1994-2005

Electronic data base consulted during the international search (name of data base and, where practicable, search terms used)
   BIOSIS/MEDLINE/WPIDS(STN), CA(STN), JSTPlus(JOIS)

C. DOCUMENTS CONSIDERED TO BE RELEVANT

| Category* | Citation of document, with indication, where appropriate, of the relevant passages | Relevant to claim No. |
| --- | --- | --- |
| A | Kenta MORIYASU et al., "ES Saibo Kara Dopamine Bunpitsu Saibo no Bunka Yudo to Sono Micro Capsule-ka", Polymer Preprints, Japan, 10 May, 2004 (10.05.04), Vol.53, No.1, page 1926 (III H03) | 1-16 |
| A | Yasunori YAMAZOE, et al., "ES Saibo kara Shinkei Saibo he Bunka Yudoyo Kizai no Sakusei", Polymer Preprints, Japan (2002), Vol.51, No.5, page 960 (I J18) | 1-16 |
| A | WO 2001/088100 A  (Kyowa Hakko Kogyo Co., Ltd.), 22 November, 2001 (22.11.01), (Family: none) | 1-16 |

| ☒ Further documents are listed in the continuation of Box C. | ☐ See patent family annex. |
| --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 29 August, 2005 (29.08.05) | 20 September, 2005 (20.09.05) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

**INTERNATIONAL SEARCH REPORT**

International application No.

PCT/JP2005/012472

C (Continuation).    DOCUMENTS CONSIDERED TO BE RELEVANT

| Category* | Citation of document, with indication, where appropriate, of the relevant passages | Relevant to claim No. |
|---|---|---|
| A | WO 2003/042384 A  (Kyowa Hakko Kogyo Co., Ltd.),<br>22 May, 2003 (22.05.03),<br>& EP 1452594 A1          & JP 2003-544199 A | 1-16 |
| A | WO 99/53028 A1  (SIGNAL PHARMACEUTICALS, INC.),<br>21 October, 1999 (21.10.99),<br>& US 6835567 B1          & CA 2325603 A<br>& EP 1071750 A          & JP 2002-511248 A<br>& US 2005/095701 A1 | 1-16 |
| A | JP 02-295481 A  (W.R. Grace & Co.-Conn.),<br>06 December, 1990 (06.12.90),<br>& US 5091176 A          & US 4940737 A<br>& CA 2001550 A          & EP 389786 A1 | 1-16 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0342384 A
- JP 2002053243 A

**Non-patent literature cited in the description**

- *New England Journal of Medicine,* 2001, vol. 344, 710
- *Nature Biotechnology,* 2000, vol. 18, 675
- *Proceedings of the National Academy of Sciences of the United States of America,* 2002, vol. 99, 1580
- *Nature,* 2002, vol. 418, 50
- *Nature Biotechnology,* 2002, vol. 20, 1240
- *Neuroscience Research,* 1994, vol. 19, 155
- Manipulating the Mouse Embryo - A Laboratory Manual. Methods in Enzymology. Academy Press, 1993, vol. 225
- *Focus,* 1998, vol. 20, 8
- *Nature,* 1997, vol. 385, 810
- *Science,* 1998, vol. 280, 1256
- *Tanpaku Kakusan Koso (Protein, Nucleic Acid, Enzyme,* 1999, vol. 44, 892
- *Nature Biotechnology,* 1999, vol. 17, 456
- *Nature,* 1998, vol. 394, 369
- *Nature Genetics,* 1999, vol. 22, 127
- *Proc. Natl. Acad Sci. USA,* 1999, vol. 96, 14984
- *Nature Genetics,* 2000, vol. 24, 109
- Molecular Cloning, A Laboratory Manual. Cold Spring Harbor Laboratory Press, 1989
- Current Protocols in Molecular Biology. John Wiley & Sons, 1987
- PCR Protocols. Academic Press, 1990
- *Nature,* 1990, vol. 344, 879
- *Proc. Soc. Exp. Biol. Med.,* 1965, vol. 89, 362
- *Exp. Cell Res.,* 1961, vol. 25, 41
- *N.Y. Academy of Sciences,* 1957, vol. 5, 303
- *Virology,* 1962, vol. 16, 147
- *J. National Cancer Inst.,* 1972, vol. 49, 1705
- *In Vitro,* 1970, vol. 9, 6
- *Proc. Soc. Exp. Biol. Med.,* 1950, vol. 73, 1
- *Science,* 1959, vol. 130, 432
- *Nature New Biology,* 1971, vol. 230, 310
- *Virology,* 1959, vol. 8, 396
- *Exp. Cell Res.,* 1963, vol. 29, 515
- *Proc. Natl. Acad. Sci. USA,* 1965, vol. 53, 288
- *J. A. M A.,* 1967, vol. 199, 519
- *Methods in Med. Res.,* 1964, vol. 10
- *Proc. Soc. Exp. Biol. Med.,* 1959, vol. 100, 115
- *Exp. Cell Res.,* 1971, vol. 69, 106
- *Exp. Cell Res.,* 1974, vol. 89, 139
- Preparation of Mutant Mice Using Embryonic Stem Cell. Methods in Enzymology. Academic Press, 1993, vol. 225
- **M.D. GOLDSBOROUGH et al.** *Focus,* 1998, vol. 20, 8
- **S. HOSOI et al.** *Cytotechnology,* 1991, vol. 5, S17
- **A. RIZZINO ; C. GROWLEY.** *Proc. Natl. Acad Sci. USA,* 1980, vol. 77, 457
- **S. OKABE et al.** *Mech. Dev.,* 1996, vol. 59, 89
- **G.R. MARTIN.** *Proc. Natl. Acad Sci. USA,* 1981, vol. 78, 7634
- Antibodies, A Laboratory Manual. Cold Spring Harbor Laboratory, 1988
- Monoclonal Antibodies: Principles and Practice. Acad. Press, 1993
- Antibody Engineering, A Practical Approach. IRL Press at Oxford University Press, 1996
- *Int. Immunol.,* 1998, vol. 10, 275
- *Exp. Hematol.,* 1997, vol. 25, 972
- Monoclonal Antibodies, Antibody Engineering. *J. Immunol.,* 1988, vol. 141, 2797
- Soshiki Baiyo No Gijutsu (Techniques of Tissue Culture). Asakura Shoten, 1996
- *Nature Neuroscience,* 1999, vol. 2, 1137
- *Nature Genet.,* 2000, vol. 24, 372